# FASCICULE DE BREVET EUROPEEN

(11) **EP 2 145 516 B1**
(45) Date de publication et mention de la délivrance du brevet: **15.09.2010**
(21) Numéro de dépôt: 08736178.8
(22) Date de dépôt: 14.04.2008
(51) Int. Cl.: H05K 5/02, A61B 6/14, H02G 15/013, H02G 15/04

(54) **PROCEDE DE FIXATION DE CÂBLE A UN BOÎTIER DE CIRCUIT ELECTRONIQUE**
VERFAHREN ZUR BEFESTIGUNG EINES KABELS AM GEHÄUSE EINER ELEKTRONISCHEN SCHALTUNG
METHOD FOR ATTACHING A CABLE TO THE HOUSING OF AN ELECTRONIC CIRCUIT

(30) Priorité: 17.04.2007 FR 0702780
(43) Date de publication de la demande: 20.01.2010
(73) Titulaire: E2V Semiconductors, 38120 Saint Egreve (FR)
(72) Inventeur: GIRARD, Frédéric, 38120 SAINT-EGREVE (FR)
(74) Mandataire: Guérin, Michel
(86) Numéro de dépôt international: PCT/EP2008/054475
(87) Numéro de publication internationale: WO 2008/135342

(56) Documents cités:
- EP-A- 1 699 232
- JP-A- 2000 023 973
- US-A- 5 691 539
- US-A1- 2007 081 358

## Description

L'invention concerne la fixation d'un câble électronique multiconducteurs à un circuit électronique contenu dans un boîtier étanche.

L'invention sera décrite à propos de la fixation d'un câble à un capteur radiologique dentaire intra-oral bien qu'elle soit applicable également dans d'autres cas.

Il est important que la fixation du câble soit très résistante à l'arrachage, mais aussi à la torsion du câble sur lui-même, à la flexion répétée, et aux risques de pliage du câble avec un trop faible rayon de courbure autour de sa fixation. Il est important aussi d'assurer l'étanchéité dans les contextes où il y a un risque de pénétration d'humidité (ou de toute autre atmosphère corrosive, liquide ou gazeuse) dans le boîtier, comme c'est le cas par exemple pour un capteur radiologique qui est placé dans la bouche d'un patient.

Il n'est pas facile de concevoir des procédés de fixation de câble qui permettent de tenir de manière optimale toutes ces contraintes.

Dans l'art antérieur, on a fréquemment renforcé la fixation d'un câble en effectuant un surmoulage de matière plastique souple autour de la sortie de câble, ce surmoulage englobant à la fois une partie du câble et une partie du boîtier. Ce surmoulage a le défaut d'être encombrant.

Le document US 2007/081358 A1 décrit un procédé de fixation d'un câble électrique, revêtu d'une gaine à un boîtier de circuit électronique. Le procédé selon ce document antérieur comporte les étape suivantes :
- mise en place sur l'extrémité du câble d'une douille rigide, qui présente à son extrémité une collerette débordante,
- enfoncement de la douille, dans le sens de l'axe du câble, de sorte que le corps de la douille vient s'insérer entre les conducteurs du câble et la gaine,
- insertion de l'extrémité du câble dans une ouverture du boîtier, et
- fermeture du boîtier.

L'invention a pour objet un procédé de fabrication plus efficace que ceux que l'on a proposé auparavant pour renforcer la solidité et d'autres qualités (telles que l'étanchéité) de la fixation sans pour autant augmenter l'encombrement de celle-ci, l'encombrement étant critique dans certaines applications.

Pour y parvenir, l'invention propose un procédé de fixation d'un câble électrique, revêtu d'une gaine en matériau plastique thermodéformable, à un boîtier de circuit électronique, comportant les étapes suivantes :
- mise en place sur l'extrémité dénudée du câble d'une douille rigide comprenant un corps creux dont le diamètre intérieur permet le passage des conducteurs mais non de la gaine du câble, la douille présentant à son extrémité une collerette débordante,
- mise en place de l'extrémité du câble et de la douille dans un moule chauffant comportant une partie de surface intérieure légèrement conique de diamètre inférieur à celui de la collerette et une partie cylindrique de plus grand diamètre que le grand diamètre de la partie conique,
- chauffage du moule à une température permettant de ramollir le matériau de la gaine,
- enfoncement de la douille, dans le sens de l'axe du câble, de sorte que le corps de la douille vient s'insérer entre les conducteurs du câble qu'il entoure et la gaine ramollie, la collerette de la douille repoussant le matériau de la gaine jusqu'à remplir la partie conique du moule,
- refroidissement pour solidifier la gaine, extraction de l'extrémité de câble hors du moule, l'extrémité présentant une portion de gaine conique de diamètre croissant terminée par la collerette débordante,
- insertion d'une autre extrémité du câble, par l'intérieur du boîtier, dans une ouverture du boîtier de diamètre très légèrement inférieur au diamètre le plus grand de la portion de gaine conique,
- fermeture du boîtier.

Par "forme légèrement conique" du moule, on entend le fait que le demi-angle d'ouverture de cône ne dépasse pas 0,1 radian ou même de préférence 0,05 radian.

La douille est de préférence en une matière plastique dure telle que du nylon ; elle a de préférence aussi une forme conique ou une forme conique prolongée par une partie cylindrique. Sa surface extérieure est de préférence cannelée pour présenter des rainures périphériques annulaires renforçant la résistance à l'arrachage. Une deuxième douille conique, de préférence en laiton, peut être insérée entre la première douille et les conducteurs du câble ; cette deuxième douille permet de maintenir la conicité de la première douille pendant et après le moulage, et elle contribue à la reprise de masse électrique sur une tresse métallique de blindage lorsque le câble comporte une telle tresse.

De préférence, lors du moulage et de l'enfoncement de la première douille dans la gaine ramollie, la collerette de la douille vient presser l'extrémité de gaine contre un décrochement entre la partie cylindrique et la partie conique du moule, formant ainsi à l'extrémité du câble une collerette de matière plastique qui s'ajoute à la collerette de la douille.

D'autres caractéristiques et avantages de l'invention apparaîtront à la lecture de la description détaillée qui suit et qui est faite en référence aux dessins annexés dans lesquels :
- la figure 1 représente une vue générale d'un boîtier de circuit électronique connecté à un câble multifilaire grâce au procédé selon l'invention ;
- la figure 2 représente en coupe une vue détaillée de l'extrémité du câble ;
- la figure 3 représente une vue en perspective d'une douille en partie cylindrique et en partie conique, destinée à être insérée entre les conducteurs du câble et la gaine lors de la fabrication ;
- la figure 4 représente une vue en coupe du moule chauffant destiné à conformer l'extrémité du câble ;
- la figure 5 représente une étape de mise en place de la douille à l'extrémité dénudée du câble ;
- la figure 6 représente le début d'une étape d'enfoncement de la douille dans le moule ;
- la figure 7 représente la fin de l'étape d'enfoncement de la douille dans le moule ;
- la figure 8 représente l'extraction de l'extrémité du câble hors du moule ;
- la figure 9 représente l'insertion de l'autre extrémité du câble dans l'ouverture du boîtier de circuit électronique.
- la figure 10 représente une configuration avec deux douilles, l'une en matière plastique, l'autre en laiton.

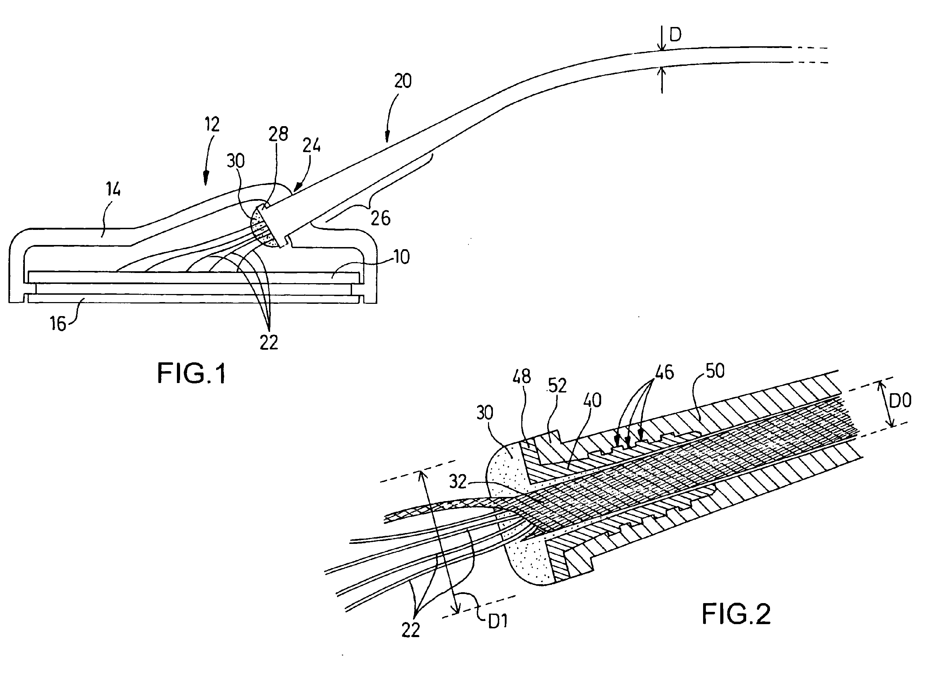

L'invention sera décrite à titre d'exemple à propos d'un capteur d'image radiologique intra-oral visible sur la figure 1. Le circuit électronique de prise d'image radiologique est symbolisé par une plaque de circuit imprimé 10 sur laquelle sont reportés classiquement divers composants non représentés, et notamment une puce de circuit intégré de prise d'image.

Le circuit électronique est monté dans un boîtier 12 comportant un capot 14 et une plaque de fermeture 16. Le capot et la plaque de fermeture peuvent être en matière plastique, ou résine composite, ou métal.

La connexion du circuit électronique avec l'extérieur est assurée par un câble multifilaire 20 dont les fils conducteurs 22 sont soudés au circuit électronique 10 à l'intérieur du boîtier. Les fils sont enfermés dans une gaine de matière plastique thermodéformable (de préférence du polyuréthane). Cette gaine passe à travers une ouverture 24 ménagée dans le capot 14 du boîtier. Les fils sortent longitudinalement à l'extrémité de la gaine, à l'intérieur du boîtier.

La gaine du câble a un diamètre généralement constant D, sauf à l'extrémité où elle est raccordée au boîtier. A cette extrémité la gaine s'évase en une forme légèrement conique dans une zone 26 : son diamètre croît en se rapprochant de l'extrémité du câble donc en se rapprochant de l'ouverture 24 du boîtier. Le diamètre naturel de la gaine à l'endroit où elle est maintenue dans l'ouverture 24 est très légèrement supérieur au diamètre de l'ouverture (par exemple supérieur d'un dixième de millimètre) ; lors de la mise en place, la gaine est forcée dans l'ouverture, son élasticité naturelle lui permettant de passer ; après sa mise en place, la gaine est appliquée très étroitement contre cette ouverture sur toute sa périphérie, assurant une étanchéité entre l'intérieur et l'extérieur du boîtier.

L'extrémité du câble comporte une collerette débordante 28, de diamètre plus large que le diamètre de l'ouverture 24, de sorte que le câble ne peut pas sortir de l'ouverture, même sous l'effet d'une forte traction depuis l'extérieur. Un dôme 30 de colle dure peut terminer la partie gainée du câble ; les fils conducteurs 22 sont maintenus par le dôme et en sortent pour être soudés sur le circuit électronique 10.

La figure 2 représente plus en détail la structure de l'extrémité conique du câble.

Le câble est représenté comme ayant plusieurs fils 22 entourés globalement par une tresse de blindage 32, mais cette tresse n'est pas obligatoire. Les fils 22 peuvent être revêtus d'une couche isolante qui est dénudée seulement à l'endroit (non représenté) de la soudure avec le circuit électronique.

Une douille rigide 40, de préférence en matière plastique dure, telle que du nylon, entoure les fils conducteurs 22 et la tresse 32, et est elle-même entourée par la gaine 50 en matière plastique thermodéformable. La douille 40 comprend de préférence des cannelures en creux et en bosses qui l'ancrent dans la gaine 50, augmentant la résistance à l'arrachage de la douille hors de la gaine (dans le sens longitudinal du câble). Les cannelures sont de préférence en forme d'anneaux circulaires autour de la douille. Elles ont de préférence une largeur (largeur des creux ou largeur des bosses) de 0,2 à 0,5 millimètre, et une profondeur de 0,2 à 0,5 millimètre.

Le dôme de colle 30 aide à la solidarisation de la douille et de la gaine, tout en maintenant en place les conducteurs 22.

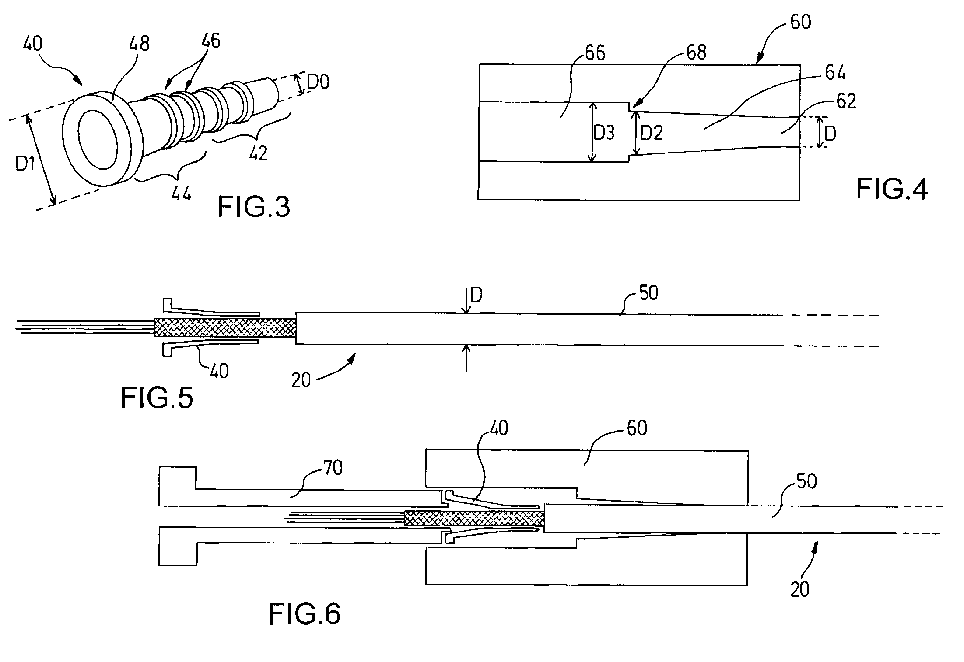

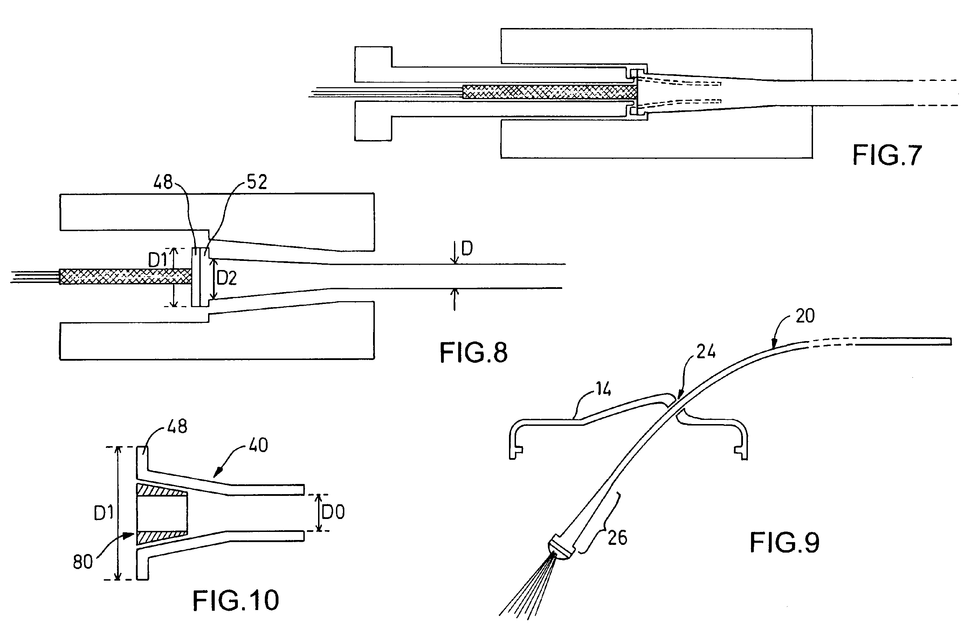

La douille 40, également visible sur la figure 3 et dans une autre forme sur la figure 10, est un corps creux qui a de préférence une partie cylindrique 42 (du côté le plus profondément enfoncé dans la gaine du câble) et une partie conique 44 (du côté le plus proche de l'extrémité du câble). La partie conique s'évase à partir de la partie cylindrique vers l'extrémité du câble. On verra plus loin (figure 10) que, dans une configuration particulière, une deuxième douille conique peut être insérée entre la première douille 40 et la tresse de blindage 32.

La matière de la douille 40 ne doit pas être thermodéformable à la température de ramollissement du matériau qui forme la gaine du câble.

La douille a de préférence, du côté de la partie cylindrique 42, un petit diamètre intérieur D0 à peine supérieur au diamètre du câble dénudé, mais inférieur au diamètre D de la gaine, afin que le câble dénudé puisse passer à l'intérieur de la douille mais que la gaine ne puisse pas y passer.

La douille est de préférence terminée par une collerette annulaire débordante 48, de diamètre D1 supérieur au diamètre extérieur de la partie conique 44. Le débordement peut être d'environ 1 millimètre.

La collerette 48 de la douille est de préférence complétée par une collerette débordante 52 de la gaine 50, de diamètre plus grand que le diamètre le plus grand de la partie conique de la gaine. Cette collerette 52 de la gaine, renforcée par la collerette 48 de la douille, s'appuie contre le rebord de l'ouverture 24 du boîtier (fig.1), à l'intérieur de celui-ci, lorsque le câble est en place. Le diamètre de la collerette 52 de la gaine est en principe égal au diamètre D1 de la collerette de la douille.

La figure 4 représente le moule de conformation de l'extrémité du câble. C'est un moule chauffant permettant de porter l'extrémité du câble à une température de ramollissement de la matière plastique formant la gaine 50.

Le moule 60 est représenté dans un état fermé ; le moule est de préférence ouvrable en deux parties, mais ce n'est pas obligatoire compte-tenu de sa forme avec un côté largement ouvert.

Le moule comporte de préférence
- une partie de surface intérieure cylindrique 62, de préférence lisse, de petit diamètre égal au diamètre D de la gaine du câble dans sa partie non élargie ;
- une partie de surface intérieure conique 64, destinée à conformer l'extrémité du câble, s'évasant légèrement depuis la partie 62, et atteignant un diamètre D2 supérieur à D ; le diamètre D2 est très légèrement supérieur (par exemple supérieur d'un dixième de millimètre) au diamètre de l'ouverture 24 du boîtier mais inférieur (d'environ 2 millimètres) au diamètre D1 de la collerette de la douille afin que la collerette ne puisse pas passer dans cette partie conique ; la surface conique 64 est de préférence lisse ;
- une partie de surface intérieure cylindrique 66 de diamètre D3 supérieur à D2, avec un décrochement 68 de hauteur (D3-D2)/2 (environ 1 millimètre) entre la partie cylindrique 66 et la partie conique 64 ; le diamètre D3 est supérieur ou égal (de préférence très légèrement supérieur) au diamètre D1 de la collerette 48 de la douille rigide 40 afin que la collerette puisse passer dans la partie cylindrique 66.

Le demi-angle d'ouverture de cône est de préférence inférieur à 0,05 radian (environ 3°). La longueur est de quelques centimètres, pour un câble de quelques millimètres de diamètre extérieur.

La figure 5 montre les premières étapes du procédé de fabrication selon l'invention : on part d'un câble 20 ayant une gaine 50 de matière thermodéformable de diamètre uniforme D ; on dénude une première extrémité du câble (à gauche sur la figure 5) ; on glisse la douille 40 par le petit diamètre de celle-ci sur l'extrémité dénudée (ici, par-dessus la tresse du câble), la douille venant en butée contre la gaine 50.

On met en place l'extrémité de câble pourvue de la douille à l'intérieur du moule 60 (figure 6).

Le câble est mis en place de telle manière qu'une partie de la gaine soit située dans la partie cylindrique 66 de diamètre D3. Il est maintenu serré dans l'autre partie cylindrique 62 de diamètre D.

On fait chauffer le moule à une température de ramollissement du matériau de la gaine. On enfonce la petite extrémité de la douille 40 dans la gaine ramollie à l'aide d'un piston 70 qui vient s'appuyer contre la collerette 48 de la douille. Le piston 70 est pourvu d'une ouverture pour recevoir l'extrémité des conducteurs du câble afin de ne pas repousser les conducteurs du câble par le piston en même temps qu'on pousse la douille.

La figure 7 représente la fin du processus d'enfoncement de la douille dans la gaine ramollie. La matière de la gaine a été repoussée par la collerette de la douille jusqu'à remplir toute la partie conique du moule en entourant le corps de la douille, et le matériau de gaine en excès par rapport à cette partie conique vient former une collerette de matière plastique, plaquée par la collerette de la douille contre le décrochement 68 du moule. Si le diamètre de la collerette de la douille est D1 très proche du diamètre D3 du moule, la collerette de matière plastique de la gaine aura pratiquement le même diamètre (D1) que la douille, conformément à ce qu'on voit sur la figure 2.

On refroidit alors le moule pour solidifier la gaine ainsi déformée, puis on extrait l'extrémité de câble hors du moule. La figure 8 représente cette extraction dans le cas d'un moule ouvrant en deux parties, qui est le cas le plus simple à mettre en oeuvre.

Le câble comporte alors une première extrémité évasée et terminée par une double collerette 48, 52. Le petit diamètre de la partie évasée est le diamètre d'origine D du câble. Le grand diamètre est le grand diamètre D2 de la surface intérieure conique 64 du moule. Et le diamètre de la collerette est D1 pour la douille, D3 presque égal à D1 pour la gaine. En dehors de la protubérance formée par la collerette, le câble est lisse, ce qui est favorable du point de vue du nettoyage.

On insère la deuxième extrémité du câble (celle qui n'a pas été conformée par le moule), par l'intérieur du boîtier de circuit électronique, dans l'ouverture 24. On tire le câble, en enfonçant à force la partie évasée de la gaine (diamètre D2) dans l'ouverture 24 (légèrement plus petite que D2) jusqu'à appliquer la collerette (48, 52) de la gaine contre le rebord intérieur de l'ouverture 24. Du fait de cet enfoncement à force, la partie évasée de la gaine établit l'étanchéité souhaitée dans l'ouverture 24.

On soude les conducteurs au circuit électronique si cela n'a pas été fait avant l'insertion.

On ferme hermétiquement le boîtier, par exemple en collant la plaque de fermeture 16 (figure 1).

Dans la réalisation décrite ci-dessus, la douille 40 possède une partie conique 44, et cette partie conique est conique aussi bien dans sa surface extérieure (en contact avec la gaine) que dans sa surface intérieure (entourant le câble dénudé). On peut prévoir dans ce cas qu'une deuxième douille rigide, de forme conique à l'extérieur mais cylindrique à l'intérieur est insérée dans la partie conique de la première douille pour maintenir la forme conique de cette dernière. La figure 10 représente l'ensemble des deux douilles. La deuxième douille 80 a un diamètre intérieur D0 juste supérieur au diamètre du câble dénudé, et un angle de conicité de surface extérieure correspondant à l'angle de conicité de la surface intérieure de la première douille. Elle est de préférence en laiton et, si le câble comporte une tresse métallique de blindage entourant les autres conducteurs, la douille vient en contact avec cette tresse et peut favoriser la prise de contact de masse avec celle-ci ; par exemple en insérant entre les deux douilles un fil conducteur destiné à être soudé au circuit électronique.

## Revendications

1. Procédé de fixation d'un câble électrique, revêtu d'une gaine en matériau plastique thermodéformable, à un boîtier (12) de circuit électronique, comportant les étapes suivantes :
- mise en place sur l'extrémité dénudée du câble d'une douille rigide (40) comprenant un corps creux dont le diamètre intérieur permet le passage des conducteurs mais non de la gaine du câble, la douille présentant à son extrémité une collerette débordante (48),
- mise en place de l'extrémité du câble et de la douille dans un moule chauffant (60) comportant une partie (64) de surface intérieure légèrement conique de diamètre inférieur à celui de la collerette et une partie cylindrique (66) de plus grand diamètre que le grand diamètre de la partie conique,
- chauffage du moule à une température permettant de ramollir le matériau de la gaine,
- enfoncement de la douille (40), dans le sens de l'axe du câble, de sorte que le corps de la douille vient s'insérer entre les conducteurs du câble qu'il entoure et la gaine ramollie, la collerette (48) de la douille repoussant le matériau de la gaine jusqu'à remplir la partie conique du moule,
- refroidissement pour solidifier la gaine, extraction de l'extrémité de câble hors du moule, l'extrémité présentant une portion de gaine conique de diamètre croissant terminée par la collerette débordante (48),
- insertion d'une autre extrémité du câble, par l'intérieur du boîtier, dans une ouverture (24) du boîtier de diamètre très légèrement inférieur au diamètre le plus grand (D2) de la portion de gaine conique,
- fermeture du boîtier.

2. Procédé selon la revendication 1, **caractérisé en ce que** la douille (40) comporte des cannelures facilitant la résistance à l'arrachage dans le sens longitudinal du câble.

3. Procédé selon l'une des revendications 1 et 2, **caractérisé en ce que** la douille (40) est en nylon.

4. Procédé selon l'une des revendications 1 à 3, **caractérisé en ce que** la douille (40) comporte une partie conique terminée du côté de son plus grand diamètre par la collerette (48).

5. Procédé selon la revendication 4, **caractérisé en ce que** la partie conique de la douille (40) est conique à l'extérieur et à l'intérieur, et une deuxième douille (80), de forme conique à l'extérieur et cylindrique à l'intérieur, est insérée à l'intérieur de la première douille (40).

6. Procédé selon la revendication 5, **caractérisé en ce que** la deuxième douille (80) est en laiton.

7. Procédé selon l'une des revendications 1 à 6, **caractérisé en ce que** la gaine du câble est en polyuréthane.

8. Procédé selon l'une des revendications 1 à 7, **caractérisé en ce que** le diamètre (D3) de la partie cylindrique (66) du moule est supérieur d'environ 2 millimètres au plus grand diamètre (D2) de la partie conique du moule.

9. Procédé selon l'une des revendications 1 à 8, **caractérisé en ce que,** lors de l'enfoncement de la douille dans la gaine ramollie, la collerette vient presser l'extrémité de gaine contre un décrochement entre la partie cylindrique et la partie conique du moule, formant ainsi une collerette de matière plastique (52) à l'extrémité du câble.

10. Procédé selon l'une des revendications 1 à 9, **caractérisé en ce que** la surface intérieure (64) de la partie conique du moule est lisse.

## Claims

1. A method of attaching an electric cable, covered with a sheath made of a thermodeformable plastic, to an electronic circuit housing (12), comprising the following steps:
- fitting onto the stripped end of the cable a rigid bushing (40) comprising a hollow body, the inside diameter of which accepts the conductors but not the sheath of the cable, the bushing having a protruding collar (48) at its end,
- fitting the end of the cable and of the bushing into a heated mold (60) comprising a slightly conical interior surface part (64) of a diameter smaller than that of the collar and a cylindrical part (66) of a diameter larger than the large diameter of the conical part,
- heating the mold to a temperature at which the material of the sheath will soften,
- driving the bushing (40) in, in the direction of the axis of the cable, so that the body of the bushing becomes inserted between the conductors of the cable that it surrounds and the softened sheath, the collar (48) of the bushing upsetting the material of the sheath to the point that it fills the conical part of the mold,
- cooling, in order to solidify the sheath, extracting the end of the cable from the mold, the end having a conical sheath portion of increasing diameter ending with the protruding collar (48),
- inserting another end of the cable, from inside the housing, through an opening (24) in the housing, the diameter of which is very slightly smaller than the largest diameter (D2) of the conical sheath portion,
- closing the housing.

2. The method as claimed in claim 1, **characterized in that** the bushing (40) is ribbed to improve the resistance of the cable to being pulled out in the longitudinal direction thereof.

3. The method as claimed in one of claims 1 and 2, **characterized in that** the bushing (40) is made of nylon.

4. The method as claimed in one of claims 1 to 3, **characterized in that** the bushing (40) comprises a conical part ending on its largest-diameter end in the collar (48).

5. The method as claimed in claim 4, **characterized in that** the conical part of the bushing (40) is conical on the outside and on the inside, and a second bushing (80), of conical shape on the outside and which is cylindrical on the inside, is inserted inside the first bushing (40).

6. The method as claimed in claim 5, **characterized in that** the second bushing (80) is made of brass.

7. The method as claimed in one of claims 1 to 6, **characterized in that** the sheath of the cable is made of polyurethane.

8. The method as claimed in one of claims 1 to 7, **characterized in that** the diameter (D3) of the cylindrical part (66) of the mold is approximately 2 millimeters larger than the largest diameter (D2) of the conical part of the mold.

9. The method as claimed in one of claims 1 to 8, **characterized in that,** while the bushing is being driven into the softened sheath, the collar presses the end of the sheath against a step between the cylindrical part and the conical part of the mold, thus forming a plastic collar (52) at the end of the cable.

10. The method as claimed in one of claims 1 to 9, **characterized in that** the interior surface (64) of the conical part of the mold is smooth.

## Patentansprüche

1. Verfahren zum Befestigen eines elektrischen Kabels, das mit einem Mantel aus einem wärmeverformbaren Kunststoffmaterial überzogen ist, an einem elektronischen Schaltungsgehäuse (12), das die folgenden Schritte beinhaltet:
- Platzieren einer starren Hülse (40) auf dem freigelegten Ende des Kabels, wobei die Hülse einen Hohlkörper aufweist, dessen Innendurchmesser die Adern, aber nicht den Mantel des Kabels durchlässt, wobei die Hülse an ihrem Ende einen Begrenzungsflansch (48) aufweist,
- Platzieren des Endes des Kabels und der Hülse in einer Heizform (60), die einen leicht konischen Innenflächenteil (64) mit einem Innendurchmesser aufweist, der kleiner ist als der des Flansches, und einen zylindrischen Teil (66) mit einem Durchmesser, der größer ist als der große Durchmesser des konischen Teils,
- Erhitzen der Form auf eine Temperatur, bei der das Material des Mantels erweicht,
- Einpressen der Hülse (40) in der Richtung der Achse des Kabels, so dass der Körper der Hülse zwischen die Leiter des von ihr umgebenen Kabels und den erweichten Mantel eingeführt wird, wobei der Flansch (48) der Hülse das Material des Mantels zurückdrückt, bis er den konischen Teil der Form ausfüllt,
- Kühlen des Mantels, um ihn zu verfestigen, Herausnehmen des Kabelendes aus der Form, wobei das Ende einen konischen Mantelabschnitt mit zunehmendem Durchmesser aufweist, der durch den Begrenzungsflansch (48) abgeschlossen wird,
- Einführen eines anderen Endes des Kabels durch das Innere des Gehäuses in eine Öffnung (24) des Gehäuses, deren Durchmessser geringfügig kleiner ist als der größere Durchmesser (D2) des konischen Mantelabschnitts,
- Schließen des Gehäuses.

2. Verfahren nach Anspruch 1, **dadurch gekennzeichnet, dass** die Hülse (40) Rillen aufweist, die den Herausziehwiderstand in Längsrichtung des Kabels verbessert.

3. Verfahren nach Anspruch 1 oder 2, **dadurch gekennzeichnet, dass** die Hülse (40) aus Nylon ist.

4. Verfahren nach einem der Ansprüche 1 bis 3, **dadurch gekennzeichnet, dass** die Hülse (40) einen konischen Teil umfasst, der auf der Seite seines größeren Durchmessers durch den Flansch (48) abgeschlossen wird.

5. Verfahren nach Anspruch 4, **dadurch gekennzeichnet, dass** der konische Teil der Hülse (40) auf der Außenseite und auf der Innenseite konisch ist, und wobei eine zweite Hülse (80), die auf der Außenseite konisch und auf der Innenseite zylindrisch ist, ins Innere der ersten Hülse (40) eingeführt wird.

6. Verfahren nach Anspruch 5, **dadurch gekennzeichnet, dass** die zweite Hülse (80) aus Messing ist.

7. Verfahren nach einem der Ansprüche 1 bis 6, **dadurch gekennzeichnet, dass** der Mantel des Kabels aus Polyurethan ist.

8. Verfahren nach einem der Ansprüche 1 bis 7, **dadurch gekennzeichnet, dass** der Durchmesser (D3) des zylindrischen Teils (66) der Form etwa um 2 Millimeter größer ist als der größere Durchmesser (D2) des konischen Teils der Form.

9. Verfahren nach einem der Ansprüche 1 bis 8, **dadurch gekennzeichnet, dass** beim Einpressen der Hülse in den erweichten Mantel der Flansch das Mantelende gegen einen Rücksprung zwischen dem zylindrischen Teil und dem konischen Teil der Form presst und so einen Flansch aus Kunststoffmaterial (52) am Ende des Kabels bildet.

10. Verfahren nach einem der Ansprüche 1 bis 9, **dadurch gekennzeichnet, dass** die Innenfläche (64) des konischen Teils der Form glatt ist.
